# EUROPEAN PATENT SPECIFICATION

(11) **EP 1 486 488 B1**
(45) Date of publication and mention of the grant of the patent: **28.10.2009**
(21) Application number: 03090177.1
(22) Date of filing: 10.06.2003
(51) Int. Cl.: C07D 213/75, C07D 215/38, C07C 323/52, C07C 233/15, C07C 233/87, A61K 31/196, A61K 31/44, A61K 31/47, A61P 35/00, A61P 3/10

(54) **Use of a compound of formula I for making a pharmaceutical composition**
Verwendung einer Verbingung gemäss Formel I zur Herstellung einer pharmazeutischen Zubereitung
Utilisation d'un composé de formule I pour la préparation d'une composition pharmaceutique

(43) Date of publication of application: 15.12.2004
(73) Proprietor: Biondi, Ricardo, Miguel, 66424 Homburg (DE)
(72) Inventor: Biondi, Ricardo, Dr., 66424 Homburg (DE); Engel, Matthias, Dr., 66482 Zweibrücken (DE)
(74) Representative: Jungblut, Bernhard Jakob

(56) References cited:
- WO-A-01/91754
- WO-A-02/079786
- WO-A-03/016517
- DATABASE CHEMCATS Chemical abstracts service, Columbus, Ohio, US; XP002262386 & "MAYBRIDGE HTS" 14 May 2003 (2003-05-14), MAYBRIDGE PLC , TINTAGEL, CORNWALL, PL34 OHW, U.K.
- DATABASE CHEMCATS Chemical abstracts service, Columbus, Ohio, US; XP002262387 & "Maybridge HTS" 14 May 2003 (2003-05-14), MAYBRIDGE PLC , TINTAGEL, CORNWALL, PL34 OHW, U.K.

## Description

### Field of the invention.

The present invention is in the field of medicinal chemistry and relates to small molecule compounds that are protein kinase regulators, activators and inhibitors, compositions containing such compounds and methods of use.

### Background of the invention and state of the art.

The search for new therapeutic agents has been greatly aided in recent years by better understanding of the structure of enzymes and other biomolecules associated with target diseases. One important class of enzymes that has been the subject of extensive study is the protein kinases.

Protein kinases mediate intracellular signal transduction. They do this by effecting a phosphoryl transfer from a nucleoside triphosphate to a protein acceptor that is involved in a signaling pathway. There are a number of kinases and pathways through which extracellular and other stimuli cause a variety of cellular responses to occur inside the cell. Examples of such stimuli include environmental and chemical stress signals (e. g. osmotic shock, heat shock, ultraviolet radiation, bacterial endotoxin, reactive oxygen species like H₂0₂), cytokines (e.g. interleukin-1 (IL-1) and tumor necrosis factor α (TNFα)), and growth factors (e.g. insulin, insulin-like growth factor (IGF1), granulocyte macrophage colony-stimulating factor (GM-CSF), and fibroblast growth factor (FGF). An extracellular stimulus may effect one or more cellular responses related to cell growth, migration, differentiation, secretion of hormones, activation of transcription factors, muscle contraction, glucose metabolism, control of protein synthesis and regulation of cell cycle.

Many diseases are associated with abnormal cellular responses triggered by protein kinase-mediated events. These diseases include autoimmune diseases, inflammatory diseases, neurological and neurodegenerative diseases, cancer, cardiovascular diseases, allergies and asthma, Alzheimer's disease or hormone-related diseases, such as diabetes.

Accordingly, there has been a substantial effort in medicinal chemistry to find protein kinase inhibitors that are effective as therapeutic agents. Thus, protein kinases have emerged as one third of all new targets in pharmaceutical industry. The protein kinase ATP binding site is clearly a drugable site and almost all protein kinase inhibitors target this site. Importantly, a similar effort has not been evident for the development of protein kinase non-ATP competitive inhibitors. In addition, the existence of small molecule compounds that target regulatory sites on protein kinase catalytic domains have not been described, in spite of large efforts from pharmaceutical companies in developing drugs targeting this family of enzymes. One reason for the absence of such type of compounds is that small molecule compounds usually cannot promote the required conformational changes. Alternatively, the assays and analysis tools usually performed for screening and analysis of the data may not select for these compounds. Alternatively, compounds might have been disregarded as possible drugable molecules, or the regulatory sites disregarded as possible drugable sites. At any rate, the finding and demonstration that small molecule compounds can regulate protein kinase activities by interacting with regulatory sites is not obvious.

Protein phosphorylation catalysed by protein kinases is the most widely known post-translational modification found in eukaryotic systems. Within an intracellular signalling pathway, phosphorylation of an enzyme substrate can change the enzymatic activity of the given enzyme presumably by modulating intramolecular interactions. For example this could be the case of glycogen synthase, which is inhibited upon glycogen synthase kinase 3 (GSK3) phosphorylation in the absence of insulin signalling (see below). The actual mode of action of phosphates covalently bound to regulatory enzymes has been studied in several cases (1). In particular, the function of phosphates at the activation loop of many protein kinases has been analysed based on crystal structure analysis, the first one of which has been described more than 10 years ago with cyclic AMP dependent protein kinase (2). It could be suggested that small molecule compounds could be produced in such a way that they would interact with the phosphate binding sites within an enzyme and prompt the effects normally obtained upon phosphorylation. In this way, the phosphorylated state of enzymes could be mimicked and the signal transduction phosphorylation event by-passed. Nevertheless, there is no evidence in the literature that this is indeed possible. As described below and in (3), there is evidence that phosphorylated polypeptides or negatively charged polypeptides can interact with protein kinases and mimic the phosphorylated form of protein kinases. Nevertheless, evidence that any of these sites could be targeted by small molecule compounds is lacking.

AGC kinases conform a group within the protein kinase superfamily (4). AGC kinase homologues are found throughout the whole eukaryotic world. The AGC kinase group consist of 63 protein kinase domains from which 6 are predicted to be pseudogenes. AGC kinase group can be divided into several families according to their homology within the catalytic domain. Furthermore, they can be grouped and named according to the most relevant protein kinases members of each group. The AGC kinases can be divided into families according to the Protein kinome, the families are: AKT (PKB), DMPK, GRK, MAST, NDR, PDK, PKA, 4 PKC families, PKG, PKN, 4RSK families, RSKL, SGK, YANK. Each one of these families may contain subfamilies. When the tree of AGC kinases is observed, important branches within the the AGC group are formed by PKCs, PKB (AKT)/SGK, S6K/RSK/MSK, GRKs, ROCK/DMPK/LATS/NDR, MAST, and RSKL families.

AGC kinases conservation throughout evolution is reflected by their overall catalytic domain sequence conservation and importantly also by its mode of regulation. Their active conformation is regulated by the state of phosphorylation of their activation loop and to secondary phosphorylations in segments outside the catalytic domain. AGC kinases have phosphorylations within the C-terminal extension to the catalytic core, which interact with the catalytic domain. Most notably is the phosphorylation within a hydrophobic motif C-terminal to the catalytic domain which also participates in protein kinase activation. The lack of phosphorylation in this site helps to keep the protein kinase inactive in some cases, like S6K members. The mechanism by which the AGC kinases are activated upon hydrophobic motif phosphorylation appears to involve the interaction of the phosphate with a phosphate binding site, while the hydrophobic motif interacts with a hydrophobic PIF pocket (5-9). The hydrophobic PIF pocket on its own can modulate protein kinase activity (7). The role in protein kinase activation was first characterised on PDK1, by homology with PKA. By homology modelling it was found to be present and play a role on a number of AGC kinases (10). Furthermore, PDK1 and PKB crystal structures support the general existence within AGC kinases of a site homologous to the site in PKA that interacts with its Phe-X-X-PheCOOH C-terminal sequence (6,8).

It has been found that isolated polypeptides comprising a hydrophobic motif (Phe-X-X-Phe-D-Tyr/Phe) where D is a phosphorylated Ser or Thr or negatively charged residue Asp, can modulate the intrinsic activity of AGC kinases, independently on whether they possess or lack a C-terminal hydrophobic motif themselves (5,7,9). Other polypeptides that activated protein kinases PDK1, SGK1, S6K1, MSK1, RSK2, PKBalpha or PKBbeta were phosphorylated polypeptides of MW above 1990 (P-HM-RSK=2858 MW, p-HM-SGK=1990 MW, p-HM-ROK=2110 MW, p-HM-PKB= 2602 MW). For example, PIFtide, NH₂-REPRILSEEEQEMFRDFDYIADW-COOH, activated PKBbeta with a AC50=5 µM, whereas shorter versions had significantly lower affinity, reaching 75% of maximal activation of PKB at 246 µM (5). The shorter version of PIFtide described to have effect on an AGC kinase intrinsic activity, has the sequence: NH₂-MFRDFDYIADW-COOH. Thus, only molecules (polypeptides) of MW above 1478 have been proven to promote activity of any AGC kinase. This size of a compound is normally not suited for development of orally effective drugs.

The interactions between the C-terminal hydrophobic motif of PKA and its own PIF pocket can be depicted from the crystal structure of PKA (2). Similarly, numerous interactions are observed between the polypeptide sequence derived from PIFtide which activates PKB, and the catalytic domain of PKB (6). The interactions between the polypeptides and the active protein kinases which are required for binding in the active conformation are depicted in the structures, but the specific requirements for the changing of conformation or activation are poorly stablished. For example, several mutations in residues forming part of the PIF pocket have produced more active PDK1 molecules, and the molecular events that lead to this effect when mutating some residues and not others is not known. As the activation of these kinases involve important conformational changes, it is of certain doubt whether small molecule compounds could also prompt these conformational changes and regulate the activity of AGC kinases. In addition, it is unclear whether small molecule compounds could stabilise either active or inactive conformations by interaction with AGC kinase PIF pockets since the binding kinetics of PIFtide to PDK1 did not fit to simple kinetic models. Furthermore, it is not known if interactions other than those depicted in the active forms of PKA and PKB crystal structures would be required to allow the conformational change to take place. Therefore, we believe that the possibility to generate small molecule compounds targeting the PIF pocket site on AGC kinase was largely doubtful. Evidence for this is the lack of mention of this possibility in patents concerning PKB structure, where the possibility of using the ATP binding site for small molecule drug development is spelled out, but the possibility of small molecules compounds to interact with the PIF pocket (termed hydrophobic motif (HM) groove by the authors) is not mentioned (WO2003016517A2; WO2003016516A2). Most importantly, the only inhibited form of an AGC kinase that has been crystallized does not provide information on this regulatory site, since one essential part of the pocket is disordered. Therefore, it cannot be anticipated whether the inhibiting form of PKB would have a drugable pocket at the site of the PIF pocket.

AGC kinases participate in a number of signalling pathways, many of which are involved in disease states and conditions that may be improved in patients. A number of non limiting examples of conditions related to the different subfamilies are given below. Furthermore, a large list of conditions where protein kinases are involved are being grouped and continuously updated from available sources, such as the protein kinase resource (PKR) website (http://pkr.sdsc.edu). PKC family member inhibitors as sought after for a number of conditions including the treatment of cancer; virus infections, such as treatments of cytomegalovirus infections, and HIV infections (US0006291446B1, US0006107327A ), asthma (US0006103712A); pain, for example pain perception and hyperalgesia (CA0002336709A1 ); skin treatments, eg. to inhibit Langer-hans cell migration induced by the presence of an allergenic agent (AU0200218371A ); renal dysfunction, such as for treatment of renal failure, intraglomerular hypertension, inhibiting glomerolosclerosis and inhibiting glomerular intestinal fibrosis (CA0002323172A1); chronic myeloid leukaemia and cute lymphoid leukaemia (CA0002311736A1 ), treatment of sexual dysfunctions directed to a method for inducing endothelium dependent vasodilation, smooth muscle relaxation, eg. penile erection, clitoral engorgement and erection (US0006093709A ), etc. Within the subfamily including PKB and SGK, inhibitors are being searched for the treatment of disease states. SGK US0006416759B1 as an antiproliferative agent, and also for treatment of diseases related to a disturbance of ion channel activity, in particular, sodium and/or potassium channels, eg. for the regulation of blood pressure (WO2002017893A3). PKB inhibition is seeked for a number of conditions including the treatment of proliferative diseases and where apoptosis is wanted. PKB inhibitors have also been proposed to inhibiting restenosis after angioplasty (WO2003032809A2 ). PKB inhibitors can be used to promote apoptosis of rheumatoid arthritis synovial fibroblasts for the treatment of rheumatoid arthritis (WO02/083075). PKB activators and inhibitors may be used for regulating the level of mucin production; PKB activators can be used to treat mucin overproduction in several diseases including otitis media, chronic obstructive pulmonary disease, asthma and cystic fibrosis, otitis media infections, and chronic obstructive pulmonary disease caused by nontypeable Haemophilus influenzae (US2002/0151491A1); S6K/RSK subfamily can be targeted for diseases where subfamily members act downstream of MAPK signalling, for example in cancer and inflamation. Rapamycin inhibits S6K as a downstream target of mTOR; thus, inhibition of S6K may be wanted to obtain part of the responses obtained with rapamycin, as immunosupressant; also it may be used to treat cancer. The subfamily of G-protein coupled receptor kinases (GRKs) can be targeted to modulate the signal intensity of G-protein coupled receptors, which form the largest family within the human genome and are important targets in drug development and therapies. Disease state and conditions that can be treated with GRKs include neurological disorders, depression, inflammation, central nervous system states, osteoporosis, immunosuppressant, hypertension, infection, hypertension, retinitis pigmentosa, cancer, asthma, cystic fibrosis, arthritis, Alzheimer, Parkinson, rheumatoid arthreitis, and in general conditions treated with drugs which target G-protein coupled receptors,. Within the ROCK/DMPK/LATS/NDR subfamily, ROCK inhibitors are being developed as therapeutic agents for the treatment of a number of conditions, including cancer, inflamation, as immunosuppresant, a therapeutic agent of autoimune disease, an hypertension, a therapeutic agent of angina pectoris, a suppressive agent of cerebrovascular contraction, a therapeutic agent of asthma, a prophylactic agent of peripheral circulation disorder, a prophylactic agent of immature birth, a prophylactic agent of digestive tract infection, a therapeutic agent of osteoporosis, a therapeutic agent of retinopathy and a brain function improving drug (US0006218410B1).

PDK1 is being targeted for the treatment of cancer with an ATP competitive inhibitor termed UCN-01.

Activators of AGC kinases could also be used in therapeutics. For example, DMPK activators could be used for treatment of conditions where DMPK activity is reduced, including Myotonic Dystrophy. Furthermore, transient activation of PDK1 or PKB beta could be used to mimic insulin signalling action for the treatment of diabetes and states where GSK3 inhibition is required for treatment or cure of diseases. By inhibiting apoptosis, these activators may be used for treatment of diseases where apoptosis is to be avoided, such as in neurological disorders. Activators of RSK family members may compensate in part the deffects in genetic diseases such as Coffin-Lowry syndrome.Therefore, RSK2 activators may be used for treatment of mental retardations or states where neurological performace is to be enchanced. Therefore, compounds that regulate (inhibit or activate) AGC kinases are important for drug development, since they could target conditions where the AGC kinase is required to be inhibited or activated.

Modulators of AGC kinase activities could be used as a therapy for treatment of patients with degrees of mental retardation or to enhance performances where disease states are not involved.

Phosphoinositide dependent protein kinase 1 (PDK1) and Protein kinase B (Akt/PKB) are components of an intracellular signalling pathway of fundamental importance that functions to exert the effects of growth and survival factors, and which mediates the response to insulin and inflammatory signals (11). PKB enzyme is rapidly activated by PDK1 phosphorylation following stimulation of phosphoinositide 3-kinase, and generation of the lipid second messenger phosphatidylinositol-3,4,5-trisphosphate [PtdIns (3,4,5) P3].

Activated PKB phosphorylates numerous cytosolic and nuclear proteins to regulate cell metabolism, growth and survival. In the insulin signalling pathway, PKB phosphorylates GSK-3, PFK2 and mTOR, inducing glycogenesis and protein synthesis, and regulates glucose uptake by promoting the translocation of Glut4 to the plasma membrane. Cell survival and transformation are controlled by phosphorylation of BAD, caspase-9, forkhead transcription factors and IkB kinase, promoting proliferation and suppressing cell apoptosis (12). A mechanism by which PKB stimulates cell cycle progression is by phosphorylation of the CDK inhibitors p21wAF1 and p27kiP1, causing their retention in the cytoplasm (13), whereas in contrast, PKB mediates nuclear localisation of mdm2 and subsequent regulation of the mdm2/p53 pathway (14). In humans, the three isoforms of PKB are highly conserved, with a mean sequence identity of 73%, and share the same regulatory phosphorylation sites.

PKB plays an important role in the generation of human malignancy.

The enzyme is the cellular homologue of v-Akt, an oncogene of the transforming murine leukaemia virus PKB8 isolated from a mouse lymphoma (15). Viral-Akt is a fusion of the viralGag protein with the PKBalpha sequence (16).

Myristoylation of the Gag sequence targets v-Akt to the cell membrane, resulting in its constitutive phosphorylation. The genes for several isoforms of PKB are overexpressed and amplified in ovarian, prostate, pancreatic, gastric, and breast tumors (17). Compelling evidence linking PKB to oncogenesis stems from the elucidation of the mechanism of the PTEN tumour suppressor gene. PTEN is one of the most commonly mutated genes in human cancer and somatic deletions or mutations of PTEN have been identified in glioblastomas, melanoma and prostate cancers, and are associated with increased susceptibility to breast and thyroid tumours (18). PTEN negatively regulates the PI-3 kinase/PKB pathway by dephosphorylating PtdIns (3,4,5) P3 on the D-3 position, and therefore loss of PTEN activity leads to a constitutive cell survival stimulus (19,20).

Therefore, modulators (activators or inhibitors) of PDK1 or PKB could be used for treatment of diseases, e.g. the treatment of diabetes, cancer, neurodegeneration and erectile dysfunction.

By modulating PKB activity, the phosphorylation state of Glycogen synthase kinase-3 (GSK3) could be regulated. GSK-3 is a serine/threonine protein kinase comprised of isoforms that are each encoded by distinct genes (21,22). This enzyme participates in several signalling pathways important in disease and small molecule compounds are being developed as ATP competitive inhibitors. As these inhibitors are ATP competitive inhibitors, they inactivate GSK3 in all different pathways. As will be described below, GSK3 inhibition by compounds may also mimic Wnt signalling and promote proliferative disorders, e.g. colon cancer. As PKB does not affect the activity of GSK3 within Wnt signalling, modulation of PKB activity could be better used for treatment of a number of disorders which require inhibition of GSK3, without affecting Wnt signalling. Therefore, for example, PKB β activators could have the added value that they would inhibit GSK-3 downstream from PKB but not affect Wnt signalling. GSK-3 has been implicated in various diseases including diabetes, Alzheimer's disease, CNS disorders such as manic depressive disorder and neurodegenerative diseases, and cardiomyocyte hypertrophy [WO 99/65897; WO 00/38675; and Haq et al., J. Cell Biol. (2000) 151,117]. These diseases may be caused by, or result in, the abnormal operation of certain cell signaling pathways in which GSK-3 plays a role. GSK-3 has been found to phosphorylate and modulate the activity of a number of regulatory proteins. These proteins include glycogen synthase, which is the rate limiting enzyme necessary for glycogen synthesis, the microtubule associated protein Tau, the amyloid peptide, the gene transcription factor-catenin, the translation initiation factor elF2B, as well as ATP citrate lyase, axin, heat shock factor-1, c-Jun, c-Myc, c-Myb, CREB, and CEPBa. These diverse protein targets implicate GSK-3 in many aspects of cellular metabolism, proliferation, differentiation and development.In a GSK-3 mediated pathway that is relevant for the treatment of type II diabetes, insulin-induced signaling leads to cellular glucose uptake and glycogen synthesis. Along this pathway, GSK-3 is a negative regulator of the insulin-induced signal. Normally, the presence of insulin causes inhibition of GSK-3 mediated phosphorylation and deactivation of glycogen synthase.The inhibition of GSK-3 leads to increased glycogen synthesis and glucose uptake [Klein et al., PNAS, 93, 8455-9 (1996); Cross et al., Biochem. J., 303,21-26 (1994); Cohen, Biochem. Soc. Trans., 21,555-567 (1993); Massillon et al., Biochem J. 299,123-128 (1994)]. However, in a diabetic patient where the insulin response is impaired, glycogen synthesis and glucose uptake fail to increase despite the presence of relatively high blood levels of insulin. This leads to abnormally high blood levels of glucose with acute and long term effects that may ultimately result in cardiovascular disease, renal failure and blindness. In such patients, the normal insulin-induced inhibition of GSK-3 fails to occur. It has also been reported that in patients with type II diabetes, GSK-3 is overexpressed [WO 00/38675]. Therefore, inhibition of GSK-3 can mimic insulin action. GSK-3 activity has also been associated with Alzheimer's disease. This disease is characterized by the well-known P-amyloid peptide and the formation of intracellular neurofibrillary tangles. The neurofibrillary tangles contain hyperphosphorylated Tau protein where Tau is phosphorylated on abnormal sites. GSK-3 has been shown to phosphorylate these abnormal sites in cell and animal models. Furthermore, inhibition of GSK-3 has been shown to prevent hyperphosphorylation of Tau in cells [Lovestone et al., Current Biology 4, 1077-86 (1994); Brownlees et al., Neuroreport 8,3251-55 (1997)]. Therefore, it is believed that GSK-3 activity may promote generation of the neurofibrillary tangles and the progression of Alzheimer's disease. Another substrate of GSK-3 is β-catenin which is degradated after phosphorylation by GSK-3. Reduced levels of β-catenin have been reported in schizophrenic patients and have also been associated with other diseases related to an increase in neuronal cell death [Zhong et al., Nature, 395,698-702 (1998) ; Takashima et al., PNAS, 90,7789-93 (1993); Pei et al., J.Neuropathol. Exp, 56,70-78 (1997)]. As a result of the biological importance of GSK-3, there is current interest in therapeutically effective GSK-3 inhbitors. Small molecules that inhibit GSK-3 have recently been reported [WO 99/65897 (Chiron), WO02/096905 (Vertex) and WO 00/38675 (SmithKline Beecham)].

Another signalling pathway in which GSK3 participates is Wnt signalling. Wnt signalling inhibits GSK3, which in turn translates into activation of transcription factors involved in tumor development, eg in colon cancers. Thus, GSK3 inhibition by compounds to treat diabetes or neurological disorders, in time, could lead to unwanted side effects. Importantly, PKB phosphorylation does not play a role in Wnt signalling inhibition of GSK3. Therefore, a compound activating the specific PKB isoform (PKB beta), which in turn phosphorylates and inhibits GSK3 within the insulin signalling pathway may be used to mimic insulin action for the treatment of diabetes, without affecting Wnt signalling.

Objects of the Invention.

One object of the invention is to provide compounds that modulate AGC protein kinases and are suitable for the preparation of pharmaceutical compositions for oral, parenteral, topical, rectal, nasal, buccal, vaginal adminstration or via an implanted reservoir or by inhalation spray. A further object is to provide compounds that modulate AGC protein kinases having a PIF binding pocket in the N-terminal lobe of the catalytic domain. A further object is to provide compounds which activate PDK1 and inhibit PDK1 and/or PKB. A further object is to provide pharmaceutical compositions suitable for treating diseases associated with protein kinases, in particular AGC kinases, PDK1 signalling and PKB signalling, e.g. cancer or diabetes.

Definitions.

In the following text by "binding site" a site (such as an atom, a functional group of an amino acid residue or a plurality of such atoms and/or groups) in a protein kinase binding cavity is meant, which may bind to an agent compound such as a candidate modulator (e.g. inhibitor). Depending on the particular molecule in the cavity, sites may exhibit attractive or repulsive binding interactions, brought about by charge, steric considerations and the like.

By "AGC kinases" is meant any protein kinase comprising a sequence which has a sequence identity of equal to or greater than 35% at the amino acid level within residues 37-350 of the catalytic subunit of PKA (Shoji et al. , 1983). Determination of percentage sequence identity may be performed with the AMPS package as described by Barton (1994). AGC kinases are also described in detail by Hanks and Hunter, FASEB J. (1995) 9: 576, and Hardie, G. and Hanks, S.

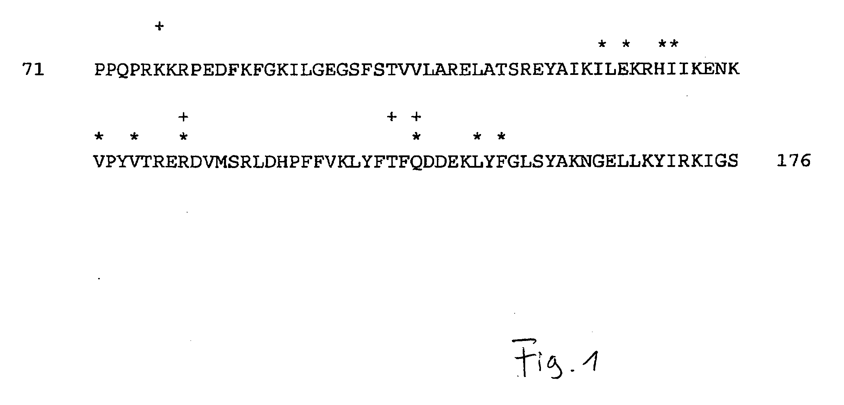

An PIF binding pocket or PIF pocket, as an example of hPDK1, is shown by the sequence according to Fig. 1. The residues marked "*" line the PIF pocket and the residues marked "+" characterize the phosphate site. In general, the following may be stated with respect to the pocket and the target proteins.

The compounds of the invention may activate or inhibit a group of protein kinases having a hydrophobic pocket in the position equivalent to the hydrophobic pocket of mouse Protein Kinase A (PKA) that is defined by residues including Lys76, Leu116, Val80 and/or Lys111 of full-length mouse PKA

The hydrophobic pocket-containing protein kinase may be the protein kinase termed 3-phosphoinositide-dependent protein kinase I (PDKI). Alternatively, it may be Serum and Glucocorticoid stimulated protein kinase (SGK), Protein Kinase B (PKB), Protein Kinase A (PKA), p70 S6 kinase, p90 RSK, PKC isoforms (for example PKCalpha, PKCbeta, PKCgamma, etc.), PRK1, PRK2, MSKI or MSK2. Hydrophobic pocket-containing protein kinases include those forming part of the AGC protein kinase group, as described by Rhodopsin and G-protein coupled receptor protein kinases, for example, also have a hydrophobic pocket as defined above and the residue equivalent to Lys76 of mouse PKA is a lysine residue.

As examples of protein kinases containing a hydrophobic pocket homologous to that of PKA, the following examples of AGC kinases are provided. The terms SGK, PKB, PKA, p70 S6 kinase (S6K), p90 RSK (RSK), PKCalpha, PKCbeta, PKCzeta or PRK2, for example, as used herein include a polypeptide (SGK, PKB, PKA, p70S6 kinase, p90 RSK, PKCalpha, PKCbeta, PKCzeta or PRK2 polypeptide) comprising the amino acid sequence identified as a SGK, PKB, PKA, p70 S6 kinase, p90 RSK, PKCalpha, PKCbeta, PKCdelta or PRK2, respectively, as identified in the relevant public database records (Table 8).

By "protein kinase having a hydrophobic pocket in the position equivalent to the hydrophobic pocket of mouse Protein Kinase A (PKA) that is defined by residues including Lys76, Leu116, Val80 and/or Lys111 of full-length mouse PKA" is meant a polypeptide having an amino acid sequence identifiable as that of a protein kinase catalytic domain, and further having a predicted or determined three-dimensional structure that includes a hydrophobic pocket corresponding to the region indicated in Knighton et al (1991) Science 253, 407-414 for PKA as interacting with C-terminal amino acids of full-length PKA, for example Phe348 and/or Phe351.

It is preferred that the protein kinase group having a hydrophobic pocket in the position equivalent to the hydrophobic pocket of mouse Protein Kinase A (PKA) that is defined by residues including Lys76, Leu116, Val 80 and/or Lys111 of full length mouse PKA is a polypeptide that is capable of interacting with a polypeptide comprising the amino acid sequence motif Phe/Tyr-Xaa-Xaa-Phe/Tyr, preferably Phe-Xaa-Xaa-Phe/Tyr, more preferably Phe-Xaa-Xaa-Phe, still more preferably Phe/Tyr-Xaa-Xaa-Phe/Tyr-Zaa-Phe/Tyr or Phe/Tyr-Xaa Xaa-Phe/Tyr-COOH, for example the polypeptide PIF or PIFtide, as defined below. It is noted that many of these hydrophobic pocket containing protein kinases will also possess a phosphate binding site equivalent to Arg131, Lys76, Thr148, Gln150 in PDK 1, which can modulate the said protein kinase activity. This was found to be the case for protein kinases PDK1, S6K1, PKBalpha, RSK2, MSK1, (Frodin et al., EMBO J. 2002 Oct 15;21(20):5396-407), and is expected to be a common feature in their respective protein kinase families including, for example all isoforms of each protein kinase studied. Therefore for a subgroup of protein kinases containing the said hydrophobic pocket, it is also preferred that the said protein kinase would interact with a polypeptide containing the amino acid sequence motif Phe/Tyr-Xaa-Xaa-Phe/Tyr-Zaa, preferably Phe-Xaa-Xaa-Phe/Tyr-Zaa, more preferably Phe-Xaa-Xaa-Phe-Zaa, still more preferably Phe/Tyr-Xaa-Xaa-Phe/Tyr-Zaa-Phe/Tyr or Phe/Tyr-Xaa-Xaa-Phe/Tyr-COOH, where Xaa is any amino acid and Zaa is an acidic amino acid (e.g. Glu or Asp) or a phosphorylated Ser or Thr.

It is preferred that the hydrophobic pocket containing protein kinase is an AGC kinase.

AGC kinase family members can be identified by performing a BLASTP search according to Altschul S.F., et al., Nucleic Acids Res. 25:3389-3402 (1997). The search sequence to be used is the prototype of the AGC kinase group, CAMP dependent protein kinase (PKA) from human origin containing the residues corresponding to the catalytic domain: 
FERIKTLGTGSFGRVMLVKHKETGNHYAMKILDKQKVVKLKQIEHTLN
EKRJLQAVNFPFLVKLEFSFKDNSNLYMVMEYVPGGEMFSHLRRIGRFS
EPHARFYAAQIVLTFEYLHSLDLIYRDLKPENLLIDQQGYIQVTDFGFAK
RVKGRTWTLCGTPEYLAPEIILSKGYNKAVDWWALGVLIYEMAAGYPP
FFADQPIQIYEKIVSGKVRFPSHFSSDLKDLLRNLLQVDLTKRFGNLKNG
VNDIKNHKWF
 NCBI BLAST program reference [PMID:9254694]: Altschul S.F., Madden T.L., Schäffer A.A., Zhang J., Zhang Z., Miller W., Lipman D.J. Gapped BLAST and PSI-BLAST: a new generation of protein database search. programs. Nucleic Acids Res. 25:3389-3402(1997). Query length: 255 AA. Date run: 2003 -06-04 12:31:58 UTC+0100 on sib-blast.unil.ch. Program: NCBI BLASTP 2.2.5 [Nov-16-2002], Database: tremblnew; trembl; swissprot, 1,132,117 sequences; 360,517,447 total letters, Swiss-Prot Release 41.10 of 30-May-2003, TrEMBL Release23.14 of 30-May-2003, TrEMBL_new of 30-May-2003

Performing a BLASTP search in this way, proteins containing similar sequences of amino acids are retrieved and characterized by a Score (bits) and an E value. It is preferred that the protein kinases with a hydrophobic motif homologous to that which interacts with the C-terminal Phe residues contains a Score higher than 140 with an E value less than 2e-33. It is further preferred that the Score is higher than 150 with an E value less than 2e-35. It is more preferred that the Score is higher than 190 and the E value less than 5e-40. It should be noted that most of AGC kinases present in databases from different organisms will be selected using this procedure. Occasionally, when the score value is lower, the parameters may select for protein kinases that may not be of the AGC kinase group but closely related families, like Aurora protein kinases, or Ca-Calmodulin dependent protein kinases, which, because of evolutionary conservations, may contain features or regulatory mechanisms (hydrophobic PIF pocket like sites in the small lobe of the catalytic domain), similar to AGC protein kinases.

It is preferred that the protein kinase has identical or conserved residues that are equivalent to Lys76, Val80, Lys111 and/or Leu116 of mouse PKA, more preferably at least Lys76 and Leu116 of mouse PKA, most preferably an identical residue equivalent to Lys76. Thus, for example, the protein kinase may have a Lys residue at the position equivalent to Lys76 of PKA and/or a Leu residue at the position equivalent to Leu116 of PKA. Lys115 and Leu155 of PDK1, for example, are equivalent to Lys76 and Leu116, respectively, of PKA. It is preferred that the protein kinase does not have an Ala at the position equivalent to Lys76 and/or a Ser, Asp or Glu at the position equivalent to Leu116 of PKA. The protein kinase may have a Val residue at the position equivalent to Leu116 of PKA, as in PRK1 and PRK2 or an Ile residue. The protein kinase may have a nonconserved residue at the position equivalent to Lys111, for example a glutamine residue and/or at the position equivalent to Va180.

The human protein kinases have been organised and tabulated. In particular, human AGC kinase group of protein kinases members has been identified from the human genome sequencing analysis (Manning G, Whyte DB, Martinez R, Hunter T, Sudarsanam S - The protein kinase complement of the human genome. Science. 2002 Dec 6; 298(5600):1912-34. Review). The information is also available at http://www.kinase.com.

Protein kinases show a conserved catalytic core, as reviewed in Johnson et al (1996) Cell, 85, 149-158 and Taylor & Radzio-Andzelm (1994) Structure 2, 345-355. This core folds into a small N-terminal lobe largely comprising antiparallel β-sheet, and a large C-terminal lobe which is mostly alpha-helical. A deep cleft at the interface between these lobes is the site of ATP binding, with the phosphate groups near the opening of the cleft.

Protein kinases also show conserved sequences within this catalytic core, and the residue equivalent to a given residue of, for example, PKA, may be identified by alignment of the sequence of the kinase with that of known kinases in such a way as to maximize the match between the sequences. The alignment may be carried out by visual inspection and/or by the use of suitable computer programs, for example the GAP program of the University of Wisconsin Genetic Computing Group, which will also allow the percent identity of the polypeptides to be calculated. The Align program (Pearson (1994) in: Methods in Molecular Biology, Computer Analysis of Sequence Data, Part II (Griffin, AM and Griffin, HG eds) pp 365-389, Humana Press, Clifton) may also be used.

Small molecule compounds are naturally occuring or synthetically accessible chemical structures having a molecular weight less than 1000, preferably less than 700, most preferably less than 550. The compounds typically have a molecular weight above 250.

Description of the invention and preferred embodiments.

The invention relates to the general finding that small molecule compounds can be activators or inhibitors of AGC protein kinases, which possess a PIF binding pocket in the N-terminal lobe of the catalytic domain. The patent describes activators of PDK1 and inhibitors of PDK1 and PKB. The invention also relates to methods of treating diseases associated with protein kinases, especially diseases associated with AGC kinases, PDK1 signalling and PKB signalling such as cancer, or diabetes.

The present invention relates, in particular, to the discovery that small molecule compounds, of less than 500 of molecular weight (MW) can regulate the activity of AGC kinases containing a PIF pocket homologous site in the small lobe of the kinase domain. In addition, compounds are described that can activate or inhibit AGC protein kinases, for example, PDK1 and PKB. The compounds described have overall good pharmacological properties. The compounds presented, or derivatives of these can be used for treatment of conditions where there is need for activating or inhibiting protein kinases of the AGC family.

The specific features of the invention are provided in the claims. The present invention relates in particular to a compound of formula I: 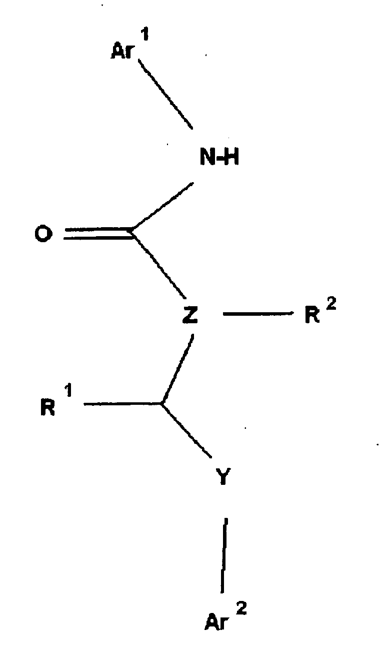 or a pharmaceutically acceptable salt thereof, wherein:
Z is selected from 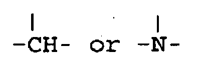
Y is selected from a valence bond or -CH₂-;
R2 is hydrogen or methyl and R1 is selected from -Q-CO₂H, -Q-CN, wherein each Q is independently selected from a valence bond or an optionally substituted C1-3 alkylidene chain, wherein one or two non-adjacent methylene units of Q are optionally and independently replaced by -O-, -S- or -NH-; or:
   R1 is hydrogen or methyl and R2 is selected from Q-CO₂H, Q-CN, wherein each Q is independently selected from a valence bond or an optionally substituted C1-3 alkylidene chain, wherein one or two non-adjacent methylene units of Q are optionally and independently replaced by -O-, -S- or -NH-;
   Ar1 and Ar2 are independently selected from a 3-10 membered monocyclic or bicyclic saturated or unsaturated cycloalkyl or aryl ring having 0-4 heteroatoms independently selected from nitrogen, oxygen, or sulphur, wherein Ar1 and/or Ar2 is optionally and independently substituted by one to four R3 groups and each R3 is independently selected from -R4, -F, -Cl, -Br, -J, -NO₂, - CN, -O-R4, -(CH₂)ₙ-O-R4 (n=1,2,3,4,5,6,7, or 8), -S-R4, - N(R4)₂, -NR4-C-O-R4, -NR4-C-O-N(R4)₂, -NR4-CO-O-R4, -COR4, -CO-O-R4, -C-O-N(R4)₂, -O-CO-N(R4)₂, -S-O-R4, -SO₂R4, -SO₂N(R4)₂, -NR4-SO₂R4, -NR4-SO₂N(R4)₂, -CO-CO-R4, or -CO-CH₂-CO-R4; wherein each R4 is independently selected from hydrogen, or from an C1-6 aliphatic group. with the exception of 5-(3-chloro-4-methylanilino)-5-oxo-3-phenylpentanic acid
and
5-(2-fluoro-4-iodoanilino)-5-oxo-3-phenylpentanoic acid. In case of Ar1 and/or Ar2, these may e.g. either be: i) a 3-8 membered monocyclic, or 8-10 membered bicyclic saturated or partially unsaturated ring, ii) a 5-6 membered monocyclic or 8-10 membered bicyclic aryl ring, or iii) a 5-6 membered monocyclic or 8-10 membered bicyclic heteroaryl ring wherein the heteroatom is given or the heteroatoms are given as defined.

Preferred embodiments of the compound are **characterized in that:**
Ar2 is selected from phenyl, naphthyl, pyrimidinyl, pyridinyl, quinolinyl, or isoquinolinyl, wherein Ar2 is optionally substituted by one to four R3 groups,
Ar1 is selected from phenyl, naphthyl, pyrimidinyl, pyridinyl, quinolinyl, or isoquinolinyl, wherein Ar1 is optionally substituted by one to four R3 groups,
X is a valence bond; Z is a nitrogen; Y is CH₂; R2 is a hydrogen; and R1 is selected from QCO₂H or QCN, wherein each Q is independently selected from a valence bond or an optionally substituted C1-3 alkylidene chain, wherein one or two non-adjacent methylene units of Q are optionally and independently replaced by -O-, -S- or -NH-,
X is a valence bond; Z is CH; Y is a valence bond; R2 is a hydrogen; and R1 is selected from QCO₂H or QCN, wherein each Q is independently selected from a valence bond or an optionally substituted C1-3 alkylidene chain, wherein one or two non-adjacent methylene units of Q are optionally and independently replaced by -O-, -S- or -NH-,
X is NH; Z is CH; Y is a valence bond; R2 is a hydrogen; and R1 is selected from QCO₂H or QCN, wherein each Q is independently selected from a valence bond or an optionally substituted C1-3 alkylidene chain, wherein one or two non-adjacent methylene units of Q are optionally and independently replaced by -O-, -S- or -NH-,
X is NH; Z is CH; Y is a valence bond; R1 is a hydrogen; and R2 is selected from QCO₂H or QCN, wherein each Q is independently selected from a valence bond or an optionally substituted C1-3 alkylidene chain, wherein one or two non-adjacent methylene units of Q are optionally and independently replaced by -O-, -S- or -NH-, and/or
Ar 1 and/or Ar2 is selected from phenyl, naphthyl, pyrimidinyl, pyridinyl, quinolinyl, or isoquinolinyl, wherein the one to four substituent R3 groups are hydrogens.

The invention describes for the first time the development of small molecule compounds against AGC protein kinases, which are non ATP competitive, and can promote or inhibit the activity of AGC kinases. The invention further describes for the first time the development of small molecule compounds designed to mimic the presence of a phosphate on an enzyme. The compounds of the invention can activate or inhibit AGC kinases. The target site for the compounds of the invention is not the ATP binding site. In the preferred embodiment of the invention, the compounds target a regulatory site on the protein kinase. It is also preferred that the target site is adjacent to a phosphate binding site in the AGC protein kinase or in related AGC kinases. The compounds may bind at the small lobe of the catalytic domain delimited by beta-4, beta-5, alpha-C-helix and alpha-B-helix. The target site is the site homologous to that which interacts with the C-terminal Phe residues in PKA.

Of the multiple interactions that can be envisaged for compounds interacting with AGC kinases in a non-ATP competitive manner, we here provide evidence for features that already prompt effects on AGC kinases.

The term "alkylidene chain" refers to an optionally substituted, straight or branched carbon chain that may be fully saturated or have one or more units of unsaturation.

A combination of substituents or variables is permissible only if such a combination results in a stable or chemically feasible compound. A stable compound or chemically feasible compound is one in which the chemical structure is not substantially altered when kept at a temperature of 40 °C or less, in the absence of moisture or other chemically reactive conditions, for at least a week. Unless otherwise stated, structures depicted herein are also meant to include all stereochemical forms of the structure; i. e., the R and S configurations for each asymmetric center. Therefore, single isolated stereochemical isomers as well as enantiomeric and diastereomeric mixtures of the present compounds are within the scope of the invention.

Unless otherwise stated, structures depicted herein are also meant to include compounds which differ only in the presence of one or more isotopically enriched atoms. For example, compounds having the present structures except for the replacement of one or several hydrogen atoms by deuterium or tritium atoms, or the replacement of one or several carbon atoms by ¹³C-or ¹⁴C are within the scope of this invention.

Compounds of formula I or salts thereof may be formulated into compositions. In a preferred embodiment, the composition is a pharmaceutical composition. In one embodiment, the composition comprises an amount of the protein kinase inhibitor effective to inhibit a protein kinase, particularly Aurora-2, in a biological sample or in a patient. Compounds of this invention and pharmaceutical compositions thereof, which comprise an amount of the protein kinase inhibitor effective to treat or prevent an Aurora-2-mediated condition and a pharmaceutically acceptable carrier, adjuvant, or vehicle, may be formulated for administration to a patient. The term "pharmaceutically acceptable carrier, adjuvant, or vehicle" refers to a non-toxic carrier, adjuvant, or vehicle that may be administered to a patient, together with a compound of this invention, and which does not destroy the pharmacological activity thereof.

Another aspect of this invention relates to a method of treating or preventing an AGC kinase mediated disease with an AGC kinase inhibitor, which method comprises administering to a patient in need of such a treatment a therapeutically effective amount of a compound of formula I or a pharmaceutical composition thereof.

Another aspect of this invention relates to a method of treating or preventing an AGC kinase mediated disease with an AGC kinase activator, which method comprises administering to a patient in need of such a treatment a therapeutically effective amount of a compound of formula I or a pharmaceutical composition thereof.

In another embodiment, this invention provides a composition comprising a compound of formula I and a pharmaceutically acceptable carrier. Another aspect of this invention relates to a method of treating or preventing an AGC kinase mediated disease with an AGC kinase inhibitor, which method comprises administering to a patient in need of such a treatment a therapeutically effective amount of a compound of formula I or a pharmaceutical composition thereof.

Another aspect of this invention relates to a method of inhibiting an AGC kinase activity in a patient, which method comprises administering to the patient a compound of formula I or a composition comprising said compound.

Another aspect of this invention relates to a method of treating or preventing an PDK1-mediated diseases with a PDK1 inhibitor, which method comprises administering to a patient in need of such a treatment a therapeutically effective amount of a compound of formula I or a pharmaceutical composition thereof.The terms "PDK1-mediated disease" or "PDK1- mediated condition", as used herein, mean any disease or other deleterious condition in which PDK1 is known to play a role. The terms "PDK1-mediated disease" or "PDK1- mediated condition" also mean those diseases or conditions that are alleviated by treatment with a PDK1 inhibitor or activator. PDK1-mediated diseases or conditions include, but are not limited to, proliferative disorders, cancer, and neurodegenerative disorders, diabetes. Another aspect of the invention relates to inhibiting PDK1 activity in a biological sample or a patient, which method comprises administering to the patient a compound of formula I or a composition comprising said compound.

Another aspect of the invention relates to activating PDK1 activity in a biological sample or a patient, which method comprises administering to the patient a compound of formula I or a composition comprising said compound.

Another aspect of this invention relates to a method of treating or preventing a PKB-mediated diseases with a PKB inhibitor, which method comprises administering to a patient in need of such a treatment a therapeutically effective amount of a compound of formula I or a pharmaceutical composition thereof. The terms "PKB-mediated disease" or "PKB- mediated condition", as used herein, mean any disease or other deleterious condition in which PKB is known to play a role. The terms "PKB-mediated disease" or " PKB-mediated condition" also mean those diseases or conditions that are alleviated by treatment with a PKB inhibitor. PKB-mediated diseases or conditions include, but are not limited to, proliferative disorders, cancer, and neurodegenerative disorders. The association of PKB, also known as protein kinase AKT, with various diseases has been described [Khwaja, A., Nature, pp. 33-34,1990; Zang, Q. Y., et al, Oncogene, 19 2000; Kazuhiko, N., et al, The Journal of Neuroscience, 20 2000]. Another aspect of the invention relates to inhibiting PKB activity in a biological sample or a patient, which method comprises administering to the patient a compound of formula I or a composition comprising said compound.

Another aspect of this invention relates to a method of treating or preventing a GSK-3-mediated disease with a PKB activator, which method comprises administering to a patient in need of such a treatment a therapeutically effective amount of a compound of formula I or a pharmaceutical composition thereof.

Another aspect of this invention relates to a method of treating or preventing a GSK-3-mediated disease with a PDK1 activator, which method comprises administering to a patient in need of such a treatment a therapeutically effective amount of a compound of formula I or a pharmaceutical composition thereof. One aspect of this invention relates to a method of enhancing glycogen synthesis and/or lowering blood levels of glucose in a patient in need thereof, which method comprises administering to the patient a therapeutically effective amount of a compound of formula I or a pharmaceutical composition thereof. This method is especially useful for diabetic patients.

Another aspect of this invention relates to inhibiting the production of hyperphosphorylated Tau protein, which is useful in halting or slowing the progression of Alzheimer's disease.

Another aspect of this invention relates to a method of inhibiting GSK-3 activity in a patient, which method comprises administering to the patient a compound of formula I or a composition comprising said compound.

Another aspect relates to inhibiting GSK-3 activity in a biological sample, which method comprises contacting the biological sample with the PKB activator of formula I, or a pharmaceutical composition thereof, in an amount effective to inhibit GSK-3.

Another aspect relates to inhibiting GSK-3 activity in a biological sample, which method comprises contacting the biological sample with the PDK1 activator of formula I, or a pharmaceutical composition thereof, in an amount effective to inhibit GSK-3.

Each of the aforementioned methods directed to the inhibition of GSK-3, or the treatment of a disease alleviated thereby, is preferably carried out with a preferred compound of formula I. The terms"GSK-3-mediated disease" or "GSK-3- mediated condition", as used herein, mean any disease or other deleterious condition or state in which GSK-3 is known to play a role. Such diseases or conditions include, without limitation, diabetes, Alzheimer's disease, Huntington's Disease, Parkinson's Disease, AIDSassociated dementia, amyotrophic lateral sclerosis (AML), multiple sclerosis (MS), schizophrenia, cardiomycete hypertrophy, reperfusion/ischemia, and baldness.

Another aspect of the invention relates to inhibiting GSK-3 activity in a biological sample, which method comprises contacting the biological sample with a PKB or PDK1 activator of formula I. Another aspect of this invention relates to a method of inhibiting GSK-3 activity in a patient, which method comprises administering to the patient a compound of formula I or a composition comprising said compound.

The term "patient" includes human and veterinary subjects. The term- "biological sample", as used herein, includes, without limitation, cell cultures or extracts thereof; preparations of an enzyme suitable for in vitro assay; biopsied material obtained from a mammal or extracts thereof; and blood, saliva, urine, feces, semen, tears, or other body fluids or extracts thereof. An amount effective to inhibit or activate a protein kinase, for example PDK1, PKB, or GSK3, is an amount that causes measurable inhibition or activation of the kinase activity when compared to the activity of the enzyme in the absence of a compound. Any method may be used to determine inhibition, such as, for example, the Biological Testing Examples described below.

Pharmaceutically acceptable carriers that may be used in these pharmaceutical compositions are generally known in the art. They include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic monoor di-glycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation. The pharmaceutical compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added. Alternatively, the pharmaceutical compositions of this invention may be administered in the form of suppositories for rectal administration. These can be prepared by mixing the agent with a suitable nonirritating excipient which is solid at room temperature but liquid at rectal temperature and therefore will melt in the rectum to release the drug. Such materials include cocoa butter, beeswax and polyethylene glycols. The pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs. Topical application for the lower intestinal tract can be effected in a rectal suppository formulation (see above) or in a suitable enema formulation. Topically-transdermal patches may also be used. For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water. For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum. The pharmaceutical compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents. In addition to the compounds of this invention, pharmaceutically acceptable derivatives or prodrugs of the compounds of this invention may also be employed in compositions to treat or prevent the above-identified diseases or disorders. A "pharmaceutically acceptable derivative or prodrug" means any pharmaceutically acceptable salt, ester, salt of an ester or other derivative of a compound of this invention which, upon administration to a recipient, is capable of providing, either directly or indirectly, a compound of this invention or an inhibitoriy active metabolite or residue thereof. Particularly favored derivatives or prodrugs are those that increase the bioavailability of the compounds of this invention when such compounds are administered to a patient (e. g., by allowing an orally administered compound to be more readily absorbed into the blood) or which enhance delivery of the parent compound to a biological compartment (e. g., the brain or lymphatic system) relative to the parent species. Pharmaceutically acceptable prodrugs of the compounds of this invention include, without limitation, the following derivatives of the present compounds: esters, amino acid esters, phosphate esters, metal salts sulfonate esters, carbamates, and amides. Pharmaceutically acceptable salts of the compounds of this invention include those derived from pharmaceutically acceptable inorganic and organic acids and bases. Examples of suitable acid salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, formate, fumarate, glucoheptanoate, glycerophosphate, glycolate, hemisulfate, heptanoate, hexanoate, hydrochloride, hydrobromide, hydroiodide, 2-hydroxyethanesulfonate, lactate, maleat, malonate, methanesulfonate, 2naphthalenesulfonate, nicotinate, nitrate, oxalate, palmoate, pectinate, persulfate, 3-phenylpropionate, phosphate, picrate, pivalate, propionate, salicylate, succinate, sulfate, tartrate, thiocyanate, tosylate and undecanoate. Other acids, such as oxalic, while not in themselves pharmaceutically acceptable, may be employed in the preparation of salts useful as intermediates in obtaining the compounds of the invention and their pharmaceutically acceptable acid addition salts. Salts derived from appropriate bases include alkali metal (e.g., sodium and potassium), alkaline earth metal (e.g., magnesium), ammonium and N+ (C14 alkyl) 4 salts. This invention also envisions the quaternization of any basic nitrogen-containing groups of the compounds disclosed herein. Water or oil-soluble or dispersible products may be obtained by such quaternization. The amount of the protein kinase inhibitor/activator that may be combined with the carrier materials to produce a single dosage form will vary depending upon the patient treated and the particular mode of administration. Preferably, the compositions should be formulated so that a dosage of between 0.01-100, preferably 0.1-20, mg/kg body weight/day of the inhibitor or activator can be administered to a patient receiving these compositions. It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of the inhibitor will also depend upon the particular compound in the composition. Depending upon the particular protein kinase mediated condition to be treated or prevented, additional therapeutic agents, which are normally administered to treat or prevent that condition, may be administered together with the inhibitors or activators of this invention. For example, in the treatment of cancer other chemotherapeutic agents or other antiproliferative agents may be combined with the present compounds to treat cancer. These agents include, without limitation, adriamycin, dexamethasone, vincristine, cyclophosphamide, fluorouracil, topotecan, taxol, interferons, and platinum derivatives. Other examples of agents the inhibitors of this invention may also be combined with including, without limitation, agents for treating diabetes such as insulin or insulin analogues, in injectable or inhalation form, glitazones, alpha glucosidase inhibitors, biguanides, insulin sensitizers, and sulfonyl ureas; antiinflammatory agents such as corticosteroids, TNF blockers, IL-1 RA, azathioprine, cyclophosphamide, and sulfasalazine; immunomodulatory and immunosuppressive agents such as cyclosporin, tacrolimus, rapamycin, mycophenolate mofetil, interferons, corticosteroids, cyclophophamide, azathioprine, and sulfasalazine; neurotrophic factors such as acetylcholinesterase inhibitors, MAO inhibitors, interferons, anti convulsants, ion channel blockers, riluzole, and anti Parkinsonian agents; agents for treating cardiovascular disease such as beta-blockers, ACE inhibitors, diuretics, nitrates, calcium channel blockers, and statins; agents for treating liver disease such as corticosteroids, cholestyramine, interferons, and anti-viral agents; agents for treating blood disorders such as corticosteroids, anti-leukemic agents, and growth factors; and agents for treating immunodeficiency disorders such as gamma globulin. Those additional agents may be administered separately from the protein kinase inhibitor/activator-containing composition, as part of a multiple dosage regimen. Alternatively, those agents may be part of a single dosage form, mixed together with the protein kinase inhibitor or activator of this invention in a single composition. Compounds of this invention may exist in alternative tautomeric forms. Unless otherwise indicated, the representation of either tautomer is meant to include the other.

Following the invention is further explained by means of non-limiting examples.

### Example 1: biological testing

In general, the activity of the compounds as protein kinase inhibitors or activators may be assayed in vitro, in vivo or in a cell line. In vitro assays include assays that determine inhibition or activation of either the phosphorylation activity or ATPase activity of the activated protein kinase. Alternate in vitro assays quantitate the ability of the inhibitor to bind to the protein kinase. Compound binding may be measured by radiolabelling the compound prior to binding, isolating the inhibitor or activator/protein kinase complex and determining the amount of radiolabel bound. Alternatively, inhibitor or activator binding may be determined by running a competition experiment where new compounds are incubated with the protein kinase bound to known radioligands.

Modulation of PDK1 activity may be tested as following. A compound of interest is tested for it's ability to activate or inhibit GST-PDK1 full length or truncated mutants of PDK1. The activity and modulation of activity is measured using a standard ATPgamma[32P] assay as described earlier. The assay is performed in 20 µl containing 50 mM TRIS (pH 7.5), 10 mM MgCl₂, 100 µM ATP, mM DTT. The concentration of T308tide is 1 mM and the amount of PDK1 used is 200 ng. Reactions are carried out at room temperature. Reaction is started by addition of ATP-Mg mix and stopped with phosphoric acid after 30 min.. Stopped reactions were spotted on to phosphocellulose paper (P81, Whatman) and washed. After drying, the radioactivity associated to the band was quantified.

### Example 2: compounds of the invention and their synthesis

A number of compounds according to the invention are shown in Table 1. Following several specific examples for the synthesis of compounds according to the invention are provided. Examples for the educts that have been used are given in Tables 2 to 7. These educts are available from a number of commercial sources including the companies Chembridge Ltd. (USA), Maybridge Ltd. (UK), Chemos GmbH (Germany), Sigma-Aldrich (USA), and TOKYO KASEI Organic Chemicals (Japan).

Synthesis example 2.1 (for compound ID7 and ID8 in Table 1): One educt selected from Table 2 (6 mmol) was suspended in 10 ml solution of an acetone/water mixture (50/50, v/v) and the pH adjusted to 7.0 by addition of HCl. The solution was stirred in an ice bath. 7.5 mmol of an educt selected from No. 1 to 5 in Table 3 (7.5 mmol) was mixed with 3 ml acetone, and the mixture was added drop wise to the first mixture over 1 h, while the pH of the reaction solution pH was continuously checked and readjusted. After addition, stirring in the ice bath was continued for at least another hour. The solution was then returned to room temperature and made acidic by the addition of dilute HCl to precipitate crystals. The crystals were filtered, washed with water and recrystallized from ethyl acetate to obtain the aniline-5-oxo-3-aryl pentanoic acid derivatives. The average yield was 80%.

### Synthesis example 2.2 (applied in same manner for compounds ID I and ID2 in Table 1):

One educt selected from Table 2 (5 mmol) was suspended in 10 ml solution of acetone/water (50/50, v/v) and the pH adjusted to 7.0 by addition of HCl. The solution was stirred in an ice bath. A mixture of an educt selected from No. 6 to 11 in Table 3 (6 mmol) with 2.5 ml acetone was added drop wise over 1 hour to the first mixture, while the pH of the reaction solution pH was continuously checked and readjusted. After addition, stirring in the ice bath was continued for at least another hour. The solvent was removed by rotary evaporation. The two isomeric forms obtained, derivatives of 4-anilino-4-oxo-3-(phenylmethyl) butanoic acid and 4-anilino-4-oxo-2-(phenylmethyl) butanoic acid, were dissolved in acetonitrile/H₂0 (50:50, v/v) and separated by reversed phase hplc in several runs on a preparative Synergi Polar-RP column (Phenomenex), using as a mobile phase acetonitrile/H₂O (50:50, v/v) at 1,0 ml/min.. For each of the isomers, about 71 % of the theoretical yield was obtained on average.

### Synthesis example 2.5:

A mixture of an educt selected from Table 2 (1.13 mmol), water (0.9 ml), ethylacetate (2.1 ml) and sodium hydroxide (117 mg, 2.84 mmol) was stirred between 5-15 degrees celcius for 30 minutes. To this mixture, 2,2,2-trichloroethyl chloroformiate (342 mg, 1.58 mmol) was added over 1 hour between 5-15 degrees celsius. The mixture was stirred at room temperature for 2 hours, then the aqueous layer was separated from the ethylacetate layer. The ethylacetate layer was washed with brine (2 x 0.9 ml) and dried over magnesium sulphate (60 mg). The ethylacetate layer was collected by filtration. To this solution, heptane was added. After removing part of the solvent by distillation, the product of step I was crytallized from the solution, recovered by filtration and dried in an oven to constant weight. Product I was mixed with an educt selected from Table 6 (1.1 mmol), diisopropylethylamine (1.1 mmol), and DMSO (3.3 ml), and the mixture was heated to 55 degrees celsius and held for 1.5 hours. To this solution, ethylacetate (4-5 ml) was added. The organic layer was washed with brine (4 x 2.2 ml), and dried over magnesium sulphate. The solvent was removed by rotary evaporation, and the product was crystallized from acetonitrile (2.5 ml) at 0 degrees celcius. After collecting by filtration, the final product (N,N'-disubstituted urea derivative) was recrystallized from isopropanol and dried in vacuum to constant weight.

### Example 3: Manufacturing of a pharmaceutical composition and treatment of an adult.

### Example 3.1: cancer treatment.

The compound ID1 of Table 1 is used for making a pharmaceutical composition for the treatment of cancer. 50 mg of this compound are mixed with the following components to obtain a tablet having a total weight of 130 mg.
20 mg of corn starch as a binder
20 mg of talcum as a filler
10 mg of Polyethylenglycol (PEG) 4000-6000
15 mg of magnesium stearate as a lubricant
15 mg of sodium carboxymethyl starch as a disirtegrant.

The active ingredient is mixed with the filler and the binder to obtain an essentially homogeneous mixture. This mixture is granulated under addition of a small amount of water. The obtained granulate is mixed with the remaining components. The obtained mixture is then tabletted and the obtained tablet core is optionally provided with a coating to improve taste and/or to retard release of the active ingredient.

One to 12 tablets obtained may be adminstered per day to an adult suffering from cancer. In general, compound ID1 may be administered with a dosage of 10 to 600 mg per day.

**Table 1**

| Compound ID | Structure | Compound ID | Structure |
|---|---|---|---|
| 1 | 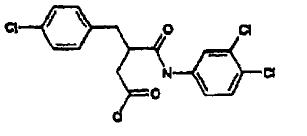 | | |
| 2 | 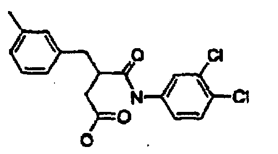 | 7 | 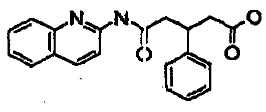 |
| | | 8 | 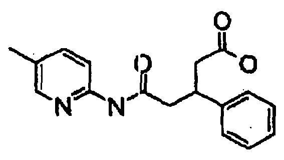 |

## Claims

1. A compound of formula I: 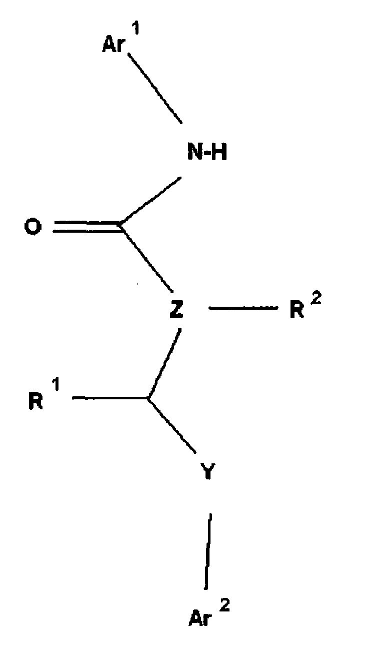 or a pharmaceutically acceptable salt thereof, wherein:
Z is selected from 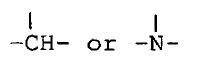
Y is selected from a valence bond or -CH₂-;
R2 is hydrogen or methyl and R1 is selected from -Q-CO₂H, -Q-CN, wherein each Q is independently selected from a valence bond or an optionally substituted C1-3 alkylidene chain, wherein one or two non-adjacent methylene units of Q are optionally and independently replaced by -O-, -S- or -NH-; or:
R1 is hydrogen or methyl and R2 is selected from Q-CO₂H, Q-CN, wherein each Q is independently selected from a valence bond or an optionally substituted C1-3 alkylidene chain, wherein one or two non-adjacent methylene units of Q are optionally and independently replaced by -O-, -S- or -NH-;
Ar1 and Ar2 are independently selected from a 3-10 membered monocyclic or bicyclic saturated or unsaturated cycloalkyl or aryl ring having 0-4 heteroatoms independently selected from nitrogen, oxygen, or sulphur, wherein Ar1 and/or Ar2 is optionally and independently substituted by one to four R3 groups and each R3 is independently selected from -R4, -F, -Cl, -Br, -J, -NO₂, - CN, -O-R4, -(CH₂)ₙ-O-R4 (n=1,2,3,4,5,6,7,or 8), -S-R4, - N(R4)₂, -NR4-C-O-R4, -NR4-C-O-N(R4)₂, -NR4-CO-O-R4, -COR4, -CO-O-R4, -C-O-N(R4)₂, -O-CO-N(R4)₂, -S-O-R4, -SO₂R4, -SO₂N(R4)₂, -NR4-SO₂R4, -NR4-SO₂N(R4)₂, -CO-CO-R4, or -CO-CH₂-CO-R4; wherein each R4 is independently selected from hydrogen, or from an C1-6 aliphatic group.
with the exception of 5-(3-chloro-4-methylanilino)-5-oxo-3-phenylpentanic acid
and
5-(2-fluoro-4-iodoanilino)-5-oxo-3-phenylpentanoic acid.

2. A compound according to claim 1, wherein Ar1 and Ar2 are independently selected from phenyl, naphtyl, pyrimidinyl, pyridinyl, quinolyl, or isoquinolyl, wherein as an option Ar1 and/or Ar2 is substituted by 1-4 R3 groups wherin R3 has the meaning explained in claim 1.

3. A compound according to claim 1 or 2, wherein Ar1 and Ar2 are independently selected from phenyl, indolyl, pyrimidinyl, pyridinyl, quinolyl, or isoquinolyl

4. A compound according to claim 1 or 2, wherein
Z is CH; Y is a valence bond; R2 is a hydrogen; and R1 is selected from QCO₂H or QCN, wherein each Q is independently selected from a valence bond or an optionally substituted C1-3 alkylidene chain, wherein one or two non-adjacent methylene units of Q are optionally and independently replaced by -O-, -S- or -NH-.

5. A compound according to claim 1 or 2, wherein
Z is CH; Y is a valence bond; R1 is a hydrogen; and R2 is selected from QCO₂H or QCN, wherein each Q is independently selected from a valence bond or an optionally substituted C1-3 alkylidene chain, wherein one or two non-adjacent methylene units of Q are optionally and independently replaced by -O-, -S- or -NH-.

6. A compound according to one of the claims 1 to 5, wherein
Z is CH,
Y is a valence bond,
R1 and R2 are independently selected from hydrogen or a group 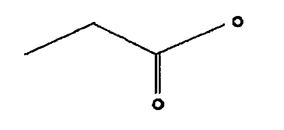
Ar1 and Ar2 are independently selected from

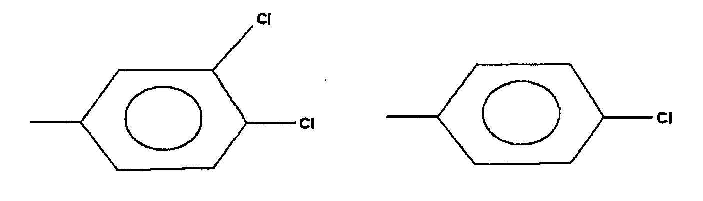

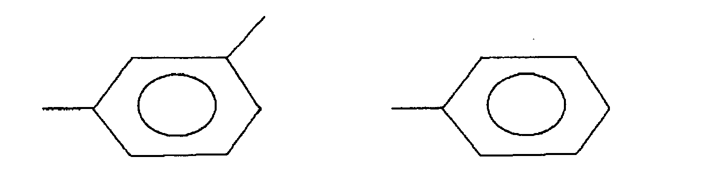

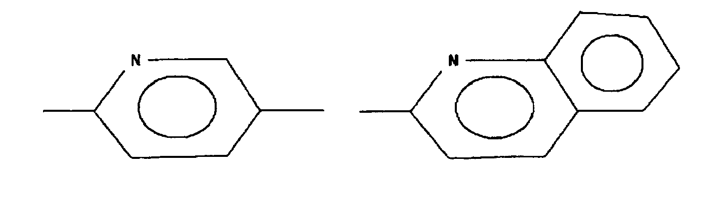

7. A compound according to one of the claims 1 to 6, wherein
Z is CH,Y is a valence bond,
R1 is hydrogen, R2 is a group 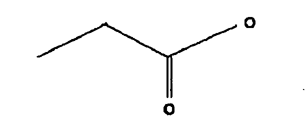
Ar1 is a group 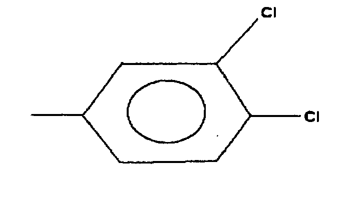
Ar2 is a group 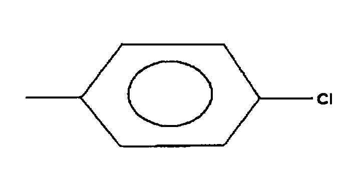 or
Z is CH,Y is a valence bond,
R1 is hydrogen, R2 is a group 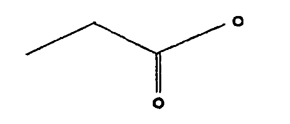
Ar1 is a group 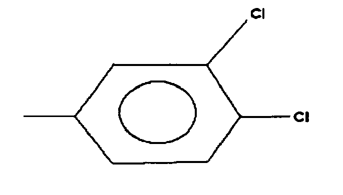
Ar2 is a group 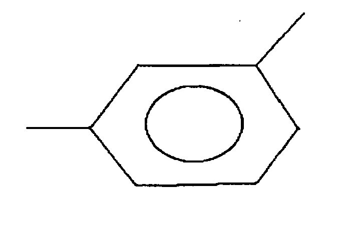 or
Z is CH,Y is a valence bond,
R2 is hydrogen, R1 is a group 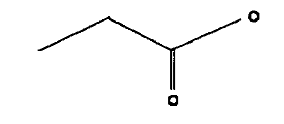
Ar1 is a group 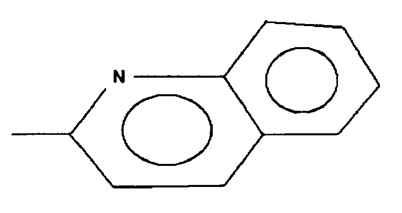
Ar2 is a group 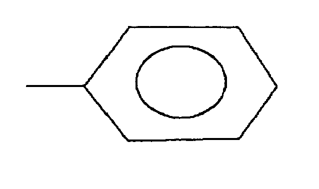 or
Z is CH,Y is a valence bond,
R2 is hydrogen, R1 is a group 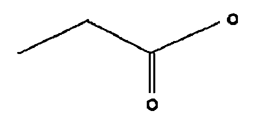
Ar1 is a group 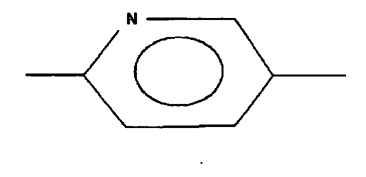
Ar2 is a group

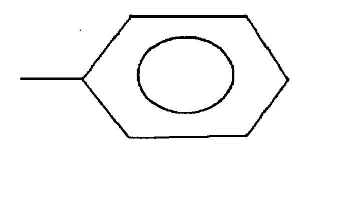

8. Use of a compound according to one of the claims 1-7 for the preparation of a pharmaceutical composition.

9. Use of a compound according to one of the claims 1-7 for the preparation of a pharmaceutical composition for the treatment of diabetes, Alzheimer's disease, Huntington's Disease, Parkinson's Disease, AIDSassociated dementia, amyotrophic lateral sclerosis (AML), multiple sclerosis (MS), schizophrenia, cardiomycete hypertrophy, ischemia, and baldness.

10. Use according to claim 8 or 9, wherein a physiologically effective dose of the compound is mixed with an pharmaceutically acceptable carrier.

11. Use of a compound according to one claims 1-7 for the in vitro modulation and/or regulation of the activity of an AGC kinase containing a PIF pocket homologuos site in the small lobe of the kinase domain, in particular for activation or inhibition of PDK1 and/or PKB.

12. Use of a compound according to one claims 1-7 for the preparation of a pharmaceutical composition for the in vivo modulation and/or regulation of the activity of an AGC kinase containing a PIF pocket homologuos site in the small lobe of the kinase domain, in particular for activation or inhibition of PDK1 and/or PKB.

13. Use of a compound according to one claims 1-7 for the preparation of a pharmaceutical composition for the prevention or treatment of a disease related to an AGC kinase, in particular PDK1 and/or PKB, having an abnormal high or low activity.

## Patentansprüche

1. Verbindung der Formel I: 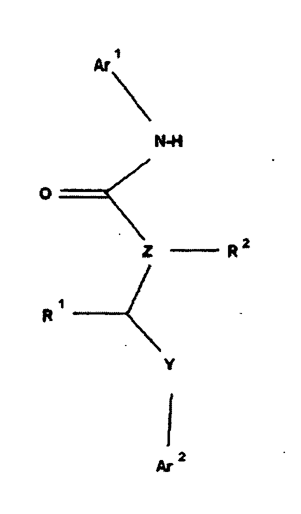 oder ein pharmazeutisch verträgliches Salz davon, wobei:
Z ausgewählt wird aus -CH- oder -N-,
Y ausgewählt wird aus einer Valenzbindung oder -CH₂-,
R2 Wasserstoff oder Methyl ist, und R1 ausgewählt wird aus -Q-CO₂H, -Q-CN, wobei jedes Q unabhängig ausgewählt wird aus einer Valenzbindung oder einer optional substituierten C1-3 Alkyliden-Kette, wobei ein oder zwei nicht-benachbarte Methylen-Einheiten von Q optional und unabhängig ersetzt werden durch -O-, -S- oder -NH-; oder
R1 Wasserstoff oder Methyl ist, und R2 ausgewählt wird aus Q-CO₂H, Q-CN, wobei jedes Q unabhängig ausgewählt wird aus einer Valenzbindung oder einer optional substituierten C1-3 Alkyliden-Kette, wobei ein oder zwei nicht-benachbarte Methylen-Einheiten von Q optional und unabhängig ersetzt werden durch -O-, -S- oder -NH-;
Ar1 und Ar2 unabhängig ausgewählt werden aus einem 3 - 10-gliedrigen monozyklischen oder bizyklischen gesättigten oder ungesättigten Zykloalkyl- oder Arylring mit 0 - 4 Heteroatomen, unabhängig ausgewählt aus Stickstoff, Sauerstoff oder Schwefel, wobei Ar1 und/oder Ar2 optional und unabhängig ersetzt werden durch eine bis vier R3-Gruppen, und jedes R3 unabhängig ausgewählt wird aus -R4, -F, -Cl, -Br, -J, -NO₂, -CN, -O-R4, -(CH₂)ₙ-O-R4 (n = 1, 2, 3, 4, 5, 6, 7 oder 8), -S-R4, -N(R4)₂, -NR4-C-O-R4, -NR4-C-O-N(R4)₂, -NR4-CO-O-R4, -CO-R4, -CO-O-R4, -C-O-N(R4)₂, -O-CO-N(R4)₂, -S-O-R4, -SO₂R4, - SO₂N(R4)₂, -NR4-SO₂R4, -NR4-SO₂N(R4)₂, -CO-CO-R4 oder -CO-CH₂-CO-R4; wobei jedes R4 unabhängig ausgewählt wird aus Wasserstoff oder aus einer C1-6-aliphatischen Gruppe,
mit der Ausnahme von 5-(3-Chlor-4-methylanilin)-5-oxo-3-Phenylpentansäure
und
5-(2-Fluor-4-iodanilin)-5-oxo-3-Phenylpentansäure.

2. Verbindung nach Anspruch 1, wobei Ar1 und Ar2 unabhängig ausgewählt werden aus Phenyl, Naphthyl, Pyrimidinyl, Pyridinyl, Quinolyl oder Isoquinolyl, wobei als Option Ar1 und/oder Ar2 substituiert ist durch 1 - 4 R3-Gruppen, wobei R3 die in Anspruch 1 erläuterte Bedeutung hat.

3. Verbindung nach Anspruch 1 oder 2, wobei Ar1 und Ar2 unabhängig ausgewählt werden aus Phenyl, Indolyl, Pyrimidinyl, Pyridinyl, Chinolinyl oder Isochinolinyl.

4. Verbindung nach Anspruch 1 oder 2, wobei Z CH ist; Y eine Valenzbindung ist; R2 Wasserstoff ist; und R1 ausgewählt wird aus QCO₂H oder QCN, wobei jedes Q unabhängig ausgewählt wird aus einer Valenzbindung oder einer optional substituierten C1-3-Alkyliden-Kette, wobei ein oder zwei nicht-benachbarte Methylen-Einheiten von Q optional und unabhängig ersetzt werden durch -O-, - S- oder -NH-.

5. Verbindung nach Anspruch 1 oder 2, wobei Z CH ist; Y eine Valenzbindung ist; R1 Wasserstoff ist; und R2 ausgewählt wird aus QCO₂H oder QCN, wobei jedes Q unabhängig ausgewählt wird aus einer Valenzbindung oder einer optional substituierten C1-3-Alkyliden-Kette, wobei ein oder zwei nicht-benachbarte Methylen-Einheiten von Q optional und unabhängig ersetzt werden durch -O-, - S- oder -NH-.

6. Verbindung nach einem der Ansprüche 1 bis 5, wobei
Z CH ist,
Y eine Valenzbindung ist,
R1 und R2 unabhängig ausgewählt werden aus Wasserstoff oder einer Gruppe 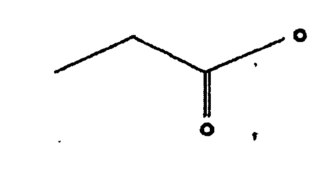
Ar1 und Ar2 unabhängig ausgewählt werden aus

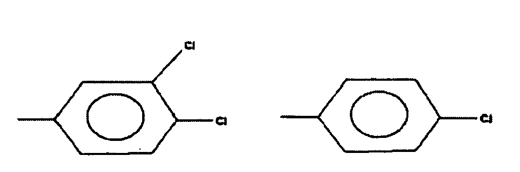

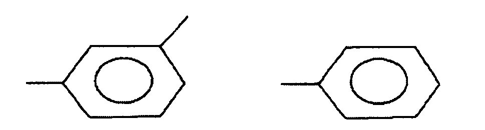

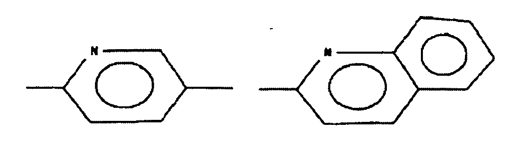

7. Verbindung nach einem der Ansprüche 1 bis 6, wobei
Z CH ist, Y eine Valenzbindung ist,
R1 Wasserstoff ist, R2 eine Gruppe ist 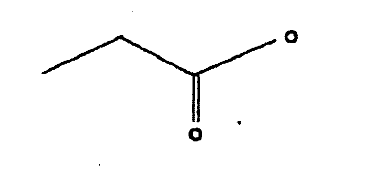
Ar1 eine Gruppe ist 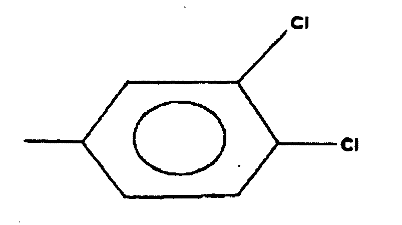
Ar2 eine Gruppe ist 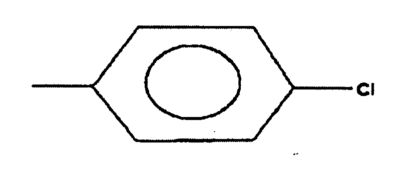 oder
Z CH ist, Y ein Valenzbindung ist,
R1 Wasserstoff ist, R2 eine Gruppe ist 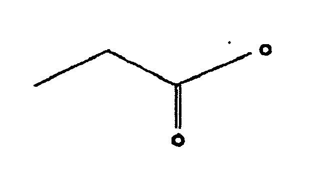
Ar1 eine Gruppe ist 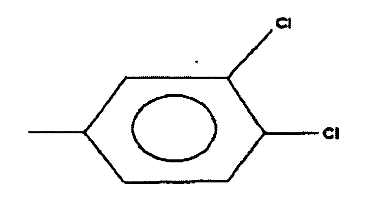
Ar2 eine Gruppe ist 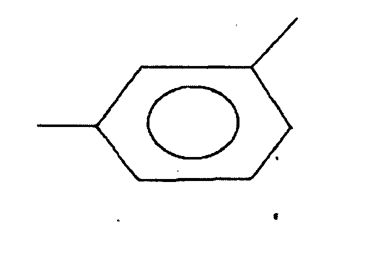 oder
Z CH ist, Y ein Valenzbindung ist,
R2 Wasserstoff ist, R1 eine Gruppe ist 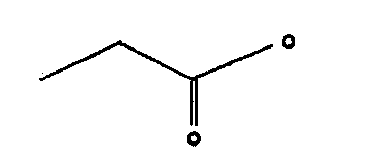
Ar1 eine Gruppe ist 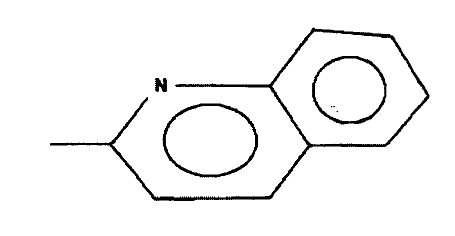
Ar2 eine Gruppe ist 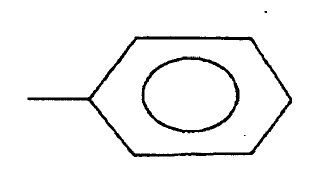 oder
Z CH ist, Y ein Valenzbindung ist,
R2 Wasserstoff ist, R1 eine Gruppe ist 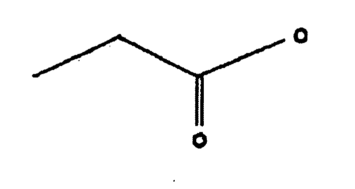
Ar1 eine Gruppe ist 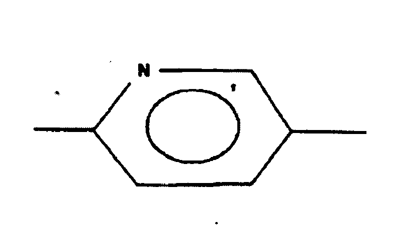
Ar2 eine Gruppe ist

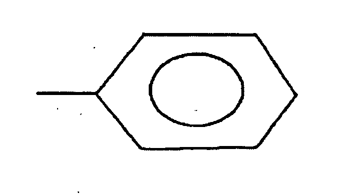

8. Verwendung einer Verbindung nach einem der Ansprüche 1 - 7 zur Herstellung einer pharmazeutischen Zusammensetzung.

9. Verwendung einer Verbindung nach einem der Ansprüche 1 - 7 zur Herstellung einer pharmazeutischen Zusammensetzung zur Behandlung von Diabetes, Alzheimer-Krankheit, Huntington-Krankheit, Parkinson-Krankheit, AIDS-induzierter Demenz, amyotropher Lateralsklerose (ALS), multipler Sklerose (MS), Schizophrenie, Kardiomyozyt-Hypertrophie, Ischämie und Kahlheit.

10. Verwendung nach Anspruch 8 oder 9, wobei eine physiologisch wirksame Dosis der Verbindung mit einem pharmazeutisch verträglichen Trägerstoff gemischt wird.

11. Verwendung einer Verbindung nach einem der Ansprüche 1 - 7 zur in vitro-Modulation und/oder -Regelung der Aktivität einer AGC-Kinase mit einer zu einer PIF-Tasche homologen Stelle im kleinen Lobe der Kinase-Domäne, insbesondere zur Aktivierung oder Inhibierung von PDK1 und/oder PKB.

12. Verwendung einer Verbindung nach einem der Ansprüche 1 - 7 zur Herstellung einer pharmazeutischen Zusammensetzung zur in vitro-Modulation und/oder -Regelung der Aktivität einer AGC-Kinase mit einer zu einer PIF-Tasche homologen Stelle im kleinen Lobe der Kinase-Domäne, insbesondere zur Aktivierung oder Inhibierung von PDK1 und/oder PKB.

13. Verwendung einer Verbindung nach einem der Ansprüche 1 - 7 zur Herstellung einer pharmazeutischen Zusammensetzung für die Prävention oder Behandlung einer mit einer AGC-Kinase zusammenhängenden Krankheit, insbesondere PDK1 und/oder PKB, mit einer abnormen hohen oder niedrigen Aktivität.

## Revendications

1. Composé de la formule I: 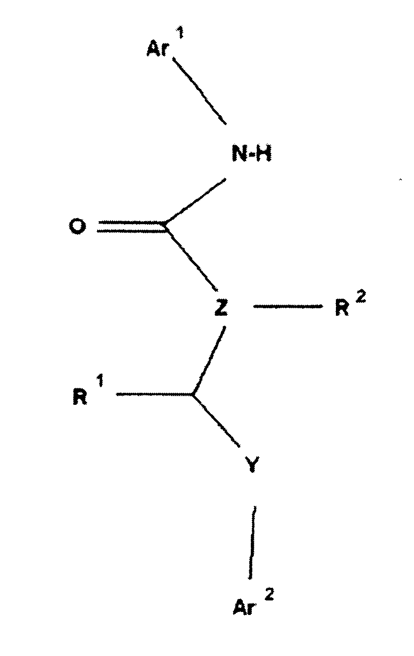 ou un sel pharmaceutiquement acceptable de celui-ci, dans lequel:
Z est choisi à partir de -CH- ou -N-,
Y est choisi à partir d'une liaison de valence ou -CH₂-,
R2 est hydrogène ou méthyle et R1 est choisi à partir de -Q-CO₂H, -Q-CN, chaque Q étant indépendamment choisi à partir d'une liaison de valence ou d'une chaîne d'alkylidènes C1-3 optionnellement substituée, une ou deux unités de méthylène de Q non voisines étant optionnellement et indépendamment remplacées par -O-, -S- ou -NH-; ou
R1 est hydrogène ou méthyle et R2 est choisi à partir de Q-CO₂H, Q-CN, chaque Q étant indépendamment choisi à partir d'une liaison de valence ou d'une chaîne d'alkylidènes C1-3 optionnellement substituée, une ou deux unités de méthylène de Q non voisines étant optionnellement et indépendamment remplacées par -O-, -S- ou -NH-;
Ar1 et Ar2 sont indépendamment choisis à partir d'un anneau de cycloalkyle ou d'aryle saturé ou non saturé monocyclique ou bicyclique à 3 - 10 membres ayant 0 - 4 hétératomes indépendamment choisis à partir de nitrogène, oxygène ou soufre, Ar1 et/ou Ar2 étant optionnellement et indépendamment substitués par un à quatre groupes R3 et chaque R3 étant indépendamment choisi à partir de -R4, -F, -Cl, -Br, -J, -NO₂, -CN, -OR4, -(CH₂)ₙ-O-R4 (n = 1, 2, 3, 4, 5, 6, 7 ou 8), -S-R4, -N(R4)₂, -NR4-C-O-R4, -NR4-C-O-N(R4)₂, - NR4-CO-O-R4, -CO-R4, -CO-O-R4, -C-O-N(R4)₂, -O-CO-N(R4)₂ -S-O-R4, -SO₂R4, -SO₂N(R4)₂, -NR4-SO₂R4, -NR4-SO₂N(R4)₂, -CO-CO-R4 ou -CO-CH₂-COR4; chaque R4 étant indépendamment choisi à partir d'hydrogène, ou à partir d'un groupe aliphatique C1-6,
à l'exception d'acide 5-(3-chloro-4-méthylaniline)-5-oxo-3-phénylpentanique
et
acide 5-(2-fluoro-4-iodoaniline)-5-oxo-3-phénylpentanique.

2. Composé selon la revendication 1, dans lequel Ar1 et Ar2 sont indépendamment choisis à partir de phényle, naphtyle, pyrimidinyle, pyridinyle, quinolyle ou isoquinolyle, Ar1 et/ou Ar2 étant optionnellement substitués par 1 - 4 groupes R3, et R3 a la signification expliquée dans la revendication 1.

3. Composé selon la revendication 1 ou 2, dans lequel Ar1 et Ar2 sont indépendamment choisis à partir de phényle, indolyle, pyrimidinyle, pyridinyle, quinolyle, ou isoquinolyle.

4. Composé selon la revendication 1 ou 2, dans lequel Z est CH; Y est une liaison de valence; R2 est hydrogène; et R1 est choisi à partir de QCO₂H ou QCN, chaque Q étant indépendamment choisi à partir d'une liaison de valence ou d'une chaîne d'alkylidènes C1-3 optionnellement substituée, une ou deux unités de méthylène de Q non voisines étant optionnellement et indépendamment remplacées par -O-, -S- ou -NH-.

5. Composé selon la revendication 1 ou 2, dans lequel Z est CH; Y est une liaison de valence; R1 est hydrogène; et R2 est choisi à partir de QCO₂H ou QCN, chaque Q étant indépendamment choisi à partir d'une liaison de valence ou d'une chaîne d'alkylidènes C1-3 optionnellement substituée, une ou deux unités de méthylène de Q non voisines étant optionnellement et indépendamment remplacées par -O-, -S- ou -NH-.

6. Composé selon une des revendications 1 à 5, dans lequel
Z est CH,
Y est une liaison de valence,
R1 et R2 sont indépendamment choisis à partir d'hydrogène ou d'un groupe 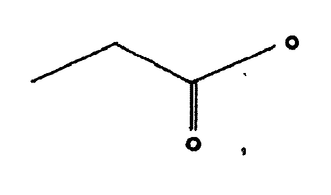
Ar1 et Ar2 sont indépendamment choisis à partir de

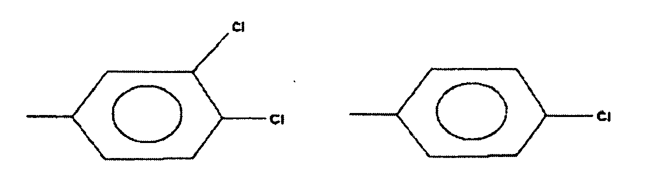

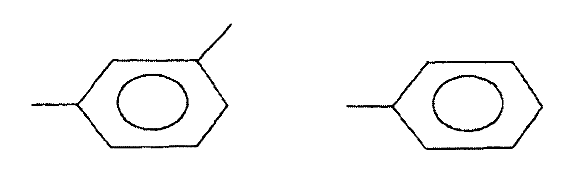

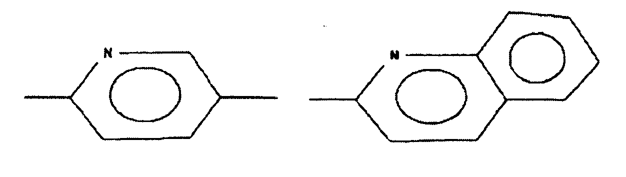

7. Composé selon une des revendications 1 à 6, dans lequel
Z est CH, Y est une liaison de valence,
R1 est hydrogène, R2 est un groupe 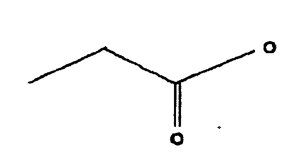
Ar1 est un groupe 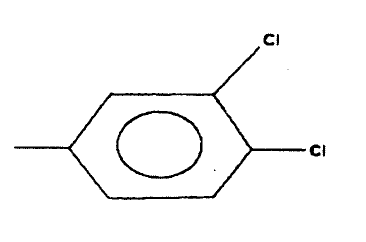
Ar2 est un groupe 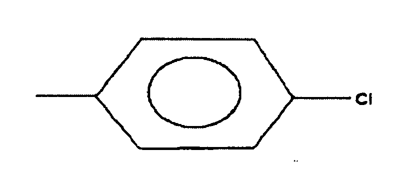 ou
Z est CH, Y est une liaison de valence,
R1 est hydrogène, R2 est un groupe 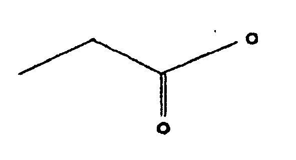
Ar1 est un groupe 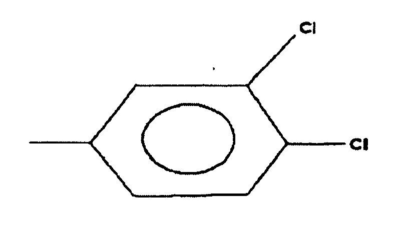
Ar2 est un groupe 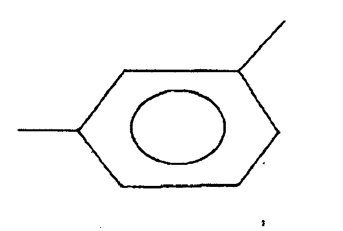 ou
Z est CH, Y est une liaison de valence,
R2 est hydrogène, R1 est un groupe 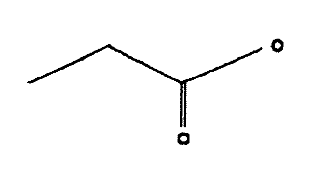
Ar1 est un groupe 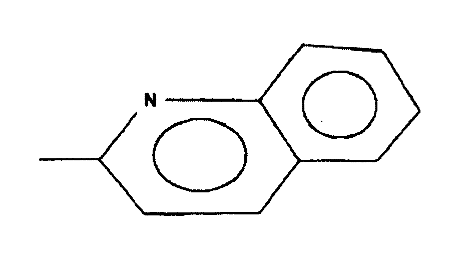
Ar2 est un groupe 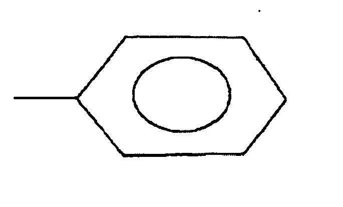 ou
Z est CH, Y est une liaison de valence,
R2 est hydrogène, R1 est un groupe 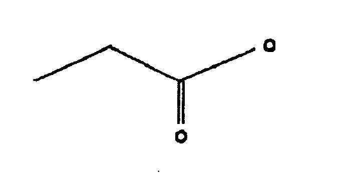
Ar1 est un groupe 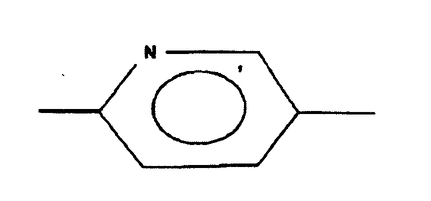
Ar2 est un groupe

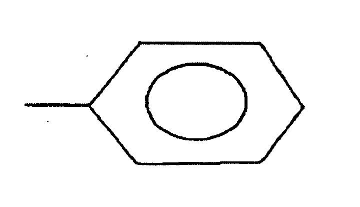

8. Utilisation d'un composé selon une des revendications 1 - 7 pour la préparation d'une composition pharmaceutique.

9. Utilisation d'un composé selon une des revendications 1 - 7 pour la préparation d'une composition pharmaceutique pour le traitement de du diabète, de la maladie d'Alzheimer, de la maladie d'Huntington, de la maladie de Parkinson, de la démence associée au SIDA, de la sclérose latérale amyotrophique (AML), de la sclérose multiple (MS), de la schizophrénie, de l'hypertrophie cardiomyocytaire, de l'ischémie et de la calvitie.

10. Utilisation selon la revendication 8 ou 9, dans lequel une dose physiologiquement efficace du composé est mélangée avec un porteur pharmaceutiquement acceptable.

11. Utilisation d'un composé selon une des revendications 1 - 7 pour la modulation et/ou régulation in vitro de l'activité d'une kinase de la famille AGC comprenant un site homologue d'une poche PIF dans le petit lobe du domaine kinase, en particulier pour l'activation ou l'inhibition de PDK1 et/ou PKB.

12. Utilisation d'un composé selon une des revendications of 1 - 7 pour la préparation d'une composition pharmaceutique pour la modulation et/ou régulation in vivo de l'activité d'une kinase de la famille AGC comprenant un site homologue d'une poche PIF dans le petit lobe du domaine kinase, en particulier pour l'activation ou l'inhibition de PDK1 et/ou PKB.

13. Utilisation d'un composé selon une des revendications 1 - 7 pour la préparation d'une composition pharmaceutique pour la prévention ou le traitement d'une maladie liée à une kinase de la famille AGC, en particulier PDK1 et/ou PKB, ayant une activité haute ou basse anormale.
